# EUROPEAN PATENT SPECIFICATION

(11) **EP 3 496 551 B1**
(45) Date of publication and mention of the grant of the patent: **02.10.2024**
(21) Application number: 17754676.9
(22) Date of filing: 11.08.2017
(51) Int. Cl.: A23K 20/163, A23L 27/30, A23L 29/30, A23L 2/60, A23D 7/005, A23L 2/52

(54) **LIQUID ALLULOSE COMPOSITION**
FLÜSSIGE ALLULOSE-ZUSAMMENSETZUNG
COMPOSITION LIQUIDE D'ALLULOSE

(30) Priority: 12.08.2016 EP 16184084
(43) Date of publication of application: 19.06.2019
(73) Proprietor: SAVANNA Ingredients GmbH, 50189 Elsdorf (DE)
(72) Inventor: KOCH, Timo Johannes, 50189 Elsdorf (DE); KIPPING, Florian, 41542 Dormagen (DE); KAUFMANN, Birgit, 50933 Köln (DE)
(74) Representative: Kutzenberger Wolff & Partner
(86) International application number: PCT/EP2017/070462
(87) International publication number: WO 2018/029351

(56) References cited:
- WO-A1-2015/075473
- WO-A1-2016/135458
- WO-A1-2017/029244
- WO-A1-2017/072569
- US-A1- 2011 237 790
- US-A1- 2014 271 748
- ORIGINAL FDA: "GRAS Notice (GRN) No. 498", 12 June 2014 (2014-06-12), XP055698086, Retrieved from the Internet <URL:http://wayback.archive-it.org/7993/20171031042510/https://www.fda.gov/downloads/Food/IngredientsPackagingLabeling/GRAS/NoticeInventory/UCM387347.pdf> [retrieved on 20200525]

## Description

The invention relates to an aqueous liquid composition comprising allulose, wherein the weight content of allulose is at least 70 wt.-%, relative to the total weight of the liquid composition; and wherein the weight content of allulose is at least 97 wt.-%, relative to the total content of all carbohydrates that are contained in the liquid composition; wherein the liquid composition has a pH value within the range of 4.0±0.5; and wherein the liquid composition has a viscosity of not more than 200 mPa·s. The invention also relates to the use of the liquid composition comprising allulose in food applications and beverage application.

Various carbohydrates including monosaccharides such as glucose and fructose as well as disaccharides such as sucrose and lactose are marketed in form of liquid compositions. These liquid compositions are nutritive sweeteners useful for the preparation of foodstuffs and beverages.

These marketed compositions, however, are not satisfactory in every respect and there is a demand for carbohydrate compositions having advantages compared to the conventional carbohydrate compositions.

It is desirable to provide carbohydrate compositions that have advantages with respect to
- sensory properties;
- optical properties;
- caloric value;
- processability in the manufacture of foodstuffs, foods or beverages;
- shipping properties;
- shelf-life and storage stability;
- adsorption of other materials.

It is desirable to have a carbohydrate composition or products prepared with the carbohydrate composition which have advantageous sensory properties when consumed, e.g. provide a better mouth feeling when chewed or swallowed and/or are less sweet. Which feeling in the mouth is more pleasant can depend on the product which is consumed. For example, a crunchier texture when chewed or swallowed can be desirable in products such as biscuits, crackers or cereals, whereas a softer texture when chewed or swallowed compared to conventional carbohydrate compositions can be desirable in e.g. dairy products or seasonings.

Besides the mouth feeling, the taste of the carbohydrate composition should not differ from natural caloric sugars (nutritional sweeteners) such as glucose and fructose as well as disaccharides such as sucrose and lactose, i.e. it should not have a bitter taste, metallic taste, astringent taste, licorice taste, a cooling taste, a lingering sweet aftertaste or show a delayed sweetness onset. Nonetheless, a lower degree of sweetness compared to invert sugar syrup can be advantageous.

Further, the carbohydrate composition should not have a pronounced laxative effect.

As the smell of a product is important for consumers, for certain applications it is desirable to provide a carbohydrate composition that does not take in odors such as odors from the packaging or products stored close to the carbohydrate composition, whereas for other applications it is desirable to provide a carbohydrate composition which takes in odors, e.g. flavors such as vanilla, orange, lavender, spices and the like.

WO 2015/075473 relates to the use of high levels of allulose in food and beverage products. WO 2016/135458 relates to allulose syrups, use of allulose syrups in the manufacture of food or beverage products, and food and beverage products made using the allulose syrups. WO 2017/072569 discloses compositions with enhanced sweetness per weight when compared to the sweetener carbohydrate or sweetener polyol component thereof, and methods for the preparation thereof.

US 2014/271748 discloses a low or zero calorie sweetener composition with sweetness synergy, providing a reduction in off-taste and a desirable temporal profile. The sweetener composition is suitable for use as a substitute for high calorie sugars. The sweetener composition is for use in food and beverage products, pharmaceutical products, nutritional products, and cosmetic products.

US 2011/237790 relates to a method of producing D-psicose crystals from a D-psicose solution by using supersaturation.

There is also a demand for carbohydrate compositions having advantageous properties with respect to shipping, storage and processability in industrial and private preparation processes of foodstuffs, beverages and/or animal feed.

There is a demand for carbohydrate compositions having a long shelf-life and storage stability e.g. due to lower contamination and/or growth of bacteria and/or fungi, especially mold, and the like. Further, there is a demand for carbohydrate compositions that keep their color and taste better than products prepared with conventional carbohydrate compositions.

Further, there is a demand to provide compositions having properties which can help to save energy in preparation processes. For example, there is a need for liquid carbohydrate compositions with a low viscosity, especially for liquid carbohydrate compositions with a high concentration of the carbohydrate. The production of liquid carbohydrate compositions having a low viscosity is more energy efficient compared to conventional carbohydrate compositions with a high viscosity. Also, the handling of a liquid carbohydrate composition with a low viscosity during production and preparation processes is easier and faster.

Furthermore, there is a demand for replacements for nutritive sweeteners, such as monosaccharides e.g. glucose and fructose as well as disaccharides e.g. sucrose and lactose, by low to zero-calorie sweeteners.

It is an object of the invention to provide carbohydrate compositions that are useful in food applications or beverage applications and that have advantages compared to the prior art.

This object has been achieved by the subject-matter of the patent claims.

It has been surprisingly found that liquid compositions comprising allulose are advantageously useful for various food applications and beverage applications, respectively.

Various properties and parameters of an aqueous liquid composition comprising allulose may become important with respect to various aspects:
- sensory properties, e.g. taste, organoleptic properties, mouth feeling, sweetness, dissolution velocity and release (velocity of development of sweetness in the mouth), and the like;
- optical properties, e.g. gloss, color, opacity, coverage, and the like;
- processability in the manufacture of foodstuffs, foods and beverages, e.g. solubility, solution rate, dust formation, dosability (gear pump, peristaltic pump, centrifugal pump, membrane pump), electrostatic charging, adhesion to surfaces, e.g. stickiness or adhesiveness (food, metal and other materials), liquid properties (viscosity, surface tension), interaction with other constituents of the foodstuff or beverage during production, pouring properties, and the like;
- storage stability and shelf-life, e.g. tendency towards microbial degradation/consumption, tendency towards oxidation, tendency towards decoloring, and the like;
- caloric value;
- segregation from other materials;
- adsorption of other materials, e.g. hygroscopicity, water content, water activity (a_{w}-value) and the like.

Due to the good water solubility of allulose and the lower sweetening capacity compared to fructose, advantageous syrups can be made from allulose. At sweetening capacities comparable of that of the corresponding fructose syrups, the allulose syrups allow for higher carbohydrate concentrations. This is particularly advantageous not only with respect to shipping costs, but also with respect to shelf-life, and storage stability, as due to the higher osmolarity of the corresponding allulose syrups, they exhibit a better stability against contamination by osmotolerant microorganisms, especially yeasts and bacteria, particularly highly osmotolerant yeasts.

It has been surprisingly found that at acidic pH (pH 2-5, preferably 2-4), aqueous liquid compositions comprising allulose are as stable against discoloration as the corresponding compositions comprising sucrose, glucose, fructose or invert sugar, although at alkaline pH aqueous liquid compositions comprising allulose have a more pronounced tendency towards discoloration. The storage stability of aqueous liquid compositions comprising allulose is a function of the pH value.

There is indication that aqueous liquid compositions comprising allulose have a surface tension that differs from the surface tension of the corresponding compositions comprising sucrose, glucose, fructose or invert sugar. This may have certain advantages for various applications, e.g. with respect to the capability of being mixed with other additives and ingredients of foodstuffs, beverages or animal feeds.

Further, there is indication that aqueous liquid compositions comprising allulose have sensory advantages, as invert sugar is too sweet for several applications, especially at high carbohydrate contents. At the same dry matter content allulose advantageously provides less sweetness in beverages than invert sugar. The aqueous liquid compositions comprising allulose are particularly advantageous for improving the taste of weight management products (e.g. dietary food and the like).

Still further, there is indication that aqueous liquid compositions comprising allulose are less sticky than the corresponding compositions comprising the corresponding amounts of sucrose, glucose, fructose or invert sugar. This is particularly advantageous with respect to processing, e.g. spraying applications.

Yet further, there is indication that allulose has a less pronounced tendency to crystallize from highly concentrated aqueous liquid compositions compared to the corresponding compositions comprising the corresponding amounts of sucrose, glucose, fructose or invert sugar. Thus, allulose remains in solution thereby providing an improved shelf-life and storage stability of aqueous liquid compositions without forming undesirable precipitates. Moreover, allulose syrups can be transported at higher concentrations even at oversaturated concentrations at ambient temperature without crystallization.

It has been surprisingly found that aqueous liquid compositions comprising allulose have a lower a_{w}-value than aqueous solutions containing the same amount of sucrose. Especially aqueous solutions with a high concentration of allulose have a considerable lower a_{w}-value than solutions containing the respective same amount of sucrose. Surprisingly, the same applies to mixtures of apple juice with allulose compared to mixtures of apple juice with sucrose, respectively.

Further, it has been surprisingly found that aqueous solutions of allulose have a lower viscosity compared to solutions containing the respective same amount of sucrose. Especially aqueous solutions with a high concentration of allulose have a lower viscosity than solutions containing the respective same amount of sucrose. Surprisingly, the same applies to mixtures of apple juice with allulose compared to mixtures of apple juice with sucrose, respectively. Even more surprisingly, the same applies to cooked mixtures of apple juice with allulose compared to mixtures of apple juice with sucrose, respectively.

There is indication, that aqueous liquid compositions comprising allulose have a prolonged shelf life due to lower bacterial contamination and do not have a tendency to decolorize. It seems that at high concentrations, allulose acts as an antioxidant. The aqueous liquid compositions comprising allulose may enhance and/or stabilize the color of other additives or ingredients of foodstuffs, beverages and animal feeds. As far as desired browning is concerned, the allulose in the aqueous liquid compositions may react in Maillard reactions and/or may be caramelized thereby providing edible brown color (e.g. burnt sugar syrup). Thus, the resultant aqueous liquid compositions comprising allulose are useful as browning syrup and browning accelerator, respectively.

Moreover, it has been surprisingly found that allulose exhibits prebiotic properties and thus can be used *inter alia* for rendering an edible composition such as a foodstuff or a beverage with prebiotic properties. Further, it has been surprisingly found that allulose has a sweetening capacity that is about 70% of that of sucrose but at the same time has no or nearly no physiological caloric value. Still further, it has been surprisingly found that allulose is not fermented by most microorganisms that are conventionally used in the production of fermented foodstuff, beverages and the like. Thus, in the presence of such microorganisms, allulose remains inert, i.e. storage stable. Accordingly, allulose can be regarded as a sweetener not exhibiting cariogenic properties. Yet further, it has been surprisingly found that allulose does not have a pronounced laxative effect. Furthermore, it has been surprisingly found that the allulose undergoes Maillard reaction with a pronounced browning effect that is comparable to that of fructose. At comparable color, a caramelized allulose has a lower sweetness than a caramelized fructose. Further, in dry caramel products, allulose provides or maintains flowability even under extreme conditions. Thus, allulose is useful as a substitute of fructose, as some people tend to develop fructose intolerance.

Furthermore, it has been surprisingly found that the color of the end product can be adjusted by the process parameters like temperature, pH value, concentration and reaction time. Due to this reason it is possible to adjust the color in the range of a clear solution up to a dark yellow solution of the end product and fit it directly to customers' demands by the process.

A first aspect of the invention relates to an aqueous liquid composition comprising allulose, wherein the weight content of allulose is at least 70 wt.-%, relative to the total weight of the liquid composition; wherein the weight content of allulose is at least 97 wt.-%, relative to the total content of all carbohydrates that are contained in the liquid composition; wherein the liquid composition has a pH value within the range of 4.0±0.5; and wherein the liquid composition has a viscosity of not more than 200 mPa·s, measured by means of a rotary viscosimeter at 23 °C at a speed of 100 rpm. The invention also relates to the use of the liquid composition comprising allulose in food and beverage applications.

Unless expressly stated otherwise, for the purpose of the specification, all percentages are weight percent.

The liquid composition according to the invention is aqueous, i.e. contains water. Preferably, water is the only liquid constituent of the composition, i.e. the only constituent that in pure form under ambient conditions is liquid as well. Thus, for example, the composition according to the invention preferably comprises no alcohol.

The aqueous composition according to the invention is liquid. While the aqueous composition according to the invention may principally be a dispersion, i.e. an emulsion and/or suspension, the aqueous composition is preferably a solution. Thus, preferably all ingredients that are contained in the aqueous composition are preferably essentially completely dissolved in the aqueous phase.

Preferably, the liquid composition according to the invention has a weight content of undissolved material of not more than 10 wt.-%, more preferably not more than 7.5 wt.-%, still more preferably not more than 5.0 wt.-%, yet more preferably not more than 3.0 wt.-%, even more preferably not more than 2.0 wt.-%, most preferably not more than 1.0 wt.-%, and in particular not more than 0.5 wt.-%, in each case relative to the total weight of the liquid composition. In a preferred embodiment, the liquid composition according to the invention contains substantially no undissolved material

Preferably, upon visual inspection with the naked eye, the liquid composition according to the invention is preferably colorless and clear.

Nonetheless, the liquid composition according to the invention may also be colored to a certain extent, e.g. slightly yellow.

Commonly, the coloring capacity of comparatively darkly colored compositions can be quantified in EBC units (European Brewery Convention).

When the liquid composition according to the invention is comparatively darkly colored, it preferably has a coloring capacity of not more than 18000 EBC, or not more than 17000 EBC, more preferably not more than 16000 EBC or not more than 15000 EBC, still more preferably of not more than 12500 EBC or not more than 10000 EBC, yet more preferably of not more than 7500 EBC or not more than 5000 EBC, even more preferably of not more than 2500 EBC or not more than 1000 EBC, most preferably of not more than 750 EBC or not more than 500 EBC, and in particular of not more than 250 EBC or not more than 100 EBC. In preferred embodiments, the liquid composition according to the invention has a coloring capacity within the range of 1000±500 EBC; 2000±1000 EBC; 3000±2000 EBC, 3000±1000 EBC; 4000±3000 EBC, 4000±2000 EBC, 4000±1000 EBC; 5000±4000 EBC, 5000±3000 EBC, 5000±2000 EBC, 5000±1000 EBC; 6000±5000 EBC, 6000±4000 EBC, 6000±3000 EBC, 6000±2000 EBC, 6000±1000 EBC; 7000±6000 EBC, 7000±5000 EBC, 7000±4000 EBC, 7000±3000 EBC, 7000±2000 EBC, 7000±1000 EBC; 8000±7000 EBC, 8000±6000 EBC, 8000±5000 EBC, 8000±4000 EBC, 8000±3000 EBC, 8000±2000 EBC, 8000±1000 EBC; 9000±8000 EBC, 9000±7000 EBC, 9000±6000 EBC, 9000±5000 EBC, 9000±4000 EBC, 9000±3000 EBC, 9000±2000 EBC, 9000±1000 EBC; 10000±8000 EBC, 10000±7000 EBC, 10000±6000 EBC, 10000±5000 EBC, 10000±4000 EBC, 10000±3000 EBC, 10000±2000 EBC, 10000±1000 EBC; 11000±7000 EBC, 11000±6000 EBC, 11000±5000 EBC, 11000±4000 EBC, 11000±3000 EBC, 11000±2000 EBC, 11000±1000 EBC; 12000±6000 EBC, 12000±5000 EBC, 12000±4000 EBC, 12000±3000 EBC, 12000±2000 EBC, 12000±1000 EBC; 13000±5000 EBC, 13000±4000 EBC, 13000±3000 EBC, 13000±2000 EBC, 13000±1000 EBC; 14000±4000 EBC, 14000±3000 EBC, 14000±2000 EBC, 14000±1000 EBC; 15000±3000 EBC, 15000±2000 EBC, 15000±1000 EBC; 16000±2000 EBC, 16000±1000 EBC; or 17000±1000.

Commonly, the coloring capacity of comparatively lightly colored compositions, e.g. only slightly yellow or yellowish compositions, can be quantified in ICUMSA units (International Commission for Uniform Methods of Sugar Analysis).

When the liquid composition according to the invention is comparatively lightly colored, it preferably has a color of not more than 1000 ICUMSA units, or not more than 900 ICUMSA units, more preferably not more than 800 ICUMSA units or not more than 700 ICUMSA units, still more preferably of not more than 600 ICUMSA units or not more than 500 ICUMSA units, yet more preferably of not more than 400 ICUMSA units or not more than 300 ICUMSA units, even more preferably of not more than 200 ICUMSA units or not more than 100 ICUMSA units, most preferably of not more than 75 ICUMSA units or not more than 50 ICUMSA units, and in particular of not more than 25 ICUMSA units or not more than 10 ICUMSA units. In preferred embodiments, the liquid composition according to the invention has a color within the range of 100±50 ICUMSA units; 200±100 ICUMSA units; 300±200 ICUMSA units, 300±100 ICUMSA units; 400±300 ICUMSA units, 400±200 ICUMSA units, 400±100 ICUMSA units; 500±400 ICUMSA units, 500±300 ICUMSA units, 500±200 ICUMSA units, 500±100 ICUMSA units; 600±500 ICUMSA units, 600±400 ICUMSA units, 600±300 ICUMSA units, 600±200 ICUMSA units, 6000±100 ICUMSA units; 700±600 ICUMSA units, 700±500 ICUMSA units, 700±400 ICUMSA units, 700±300 ICUMSA units, 700±200 ICUMSA units, 700±100 ICUMSA units; 800±700 ICUMSA units, 800±600 ICUMSA units, 800±500 ICUMSA units, 800±400 ICUMSA units, 800±300 ICUMSA units, 800±200 ICUMSA units, 800±100 ICUMSA units; 900±800 ICUMSA units, 900±700 ICUMSA units, 900±600 ICUMSA units, 900±500 ICUMSA units, 900±400 ICUMSA units, 900±300 ICUMSA units, 900±200 ICUMSA units, 900±100 ICUMSA units; 1000±800 ICUMSA units, 1000±700 ICUMSA units, 1000±600 ICUMSA units, 1000±500 ICUMSA units, 1000±400 ICUMSA units, 1000±300 ICUMSA units, 1000±200 ICUMSA units, or 1000±100 ICUMSA units.

Allulose, also referred to as psicose, is a ketohexose. For the purpose of the specification, allulose is preferably provided in form of the D-enantiomer, i.e. D-allulose (CAS no. 551-68-8), which in open chain Fischer projection has the following structure:

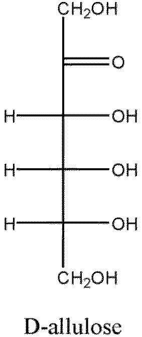

D-allulose can be present inform of the two anomers, α-D-allulose and β-D-allulose. In preferred embodiments of the powder according to the invention, at least 10 wt.-%, at least 20%, at least 30%, at least 40%, at least 50%, at least 60%, at least 70%, at least 80%, at least 90%, at least 95%, or at least 98%, at least 99% of the allulose are present in form of β-D-allulose, relative to the total weight of allulose.

The liquid composition according to the invention has a comparatively high allulose weight content of at least 70 wt.-%, relative to the total weight of the liquid composition. Thus, the liquid composition according to the invention is distinguished from e.g. beverages containing allulose due to its substantially higher allulose weight content.

The liquid composition according to the invention is preferably a syrup.

The liquid composition according to the invention is typically not devoted for intake or consumption as such, but is useful as in intermediate in the manufacture of foodstuffs, beverages or animal feeds. The liquid composition can be advantageously stored and shipped and is typically diluted in order to provide the final foodstuffs, beverages or animal feeds with the desired weight content, which is typically well below 10 wt.-%, relative to the total weight of the foodstuff, beverage and animal feed, respectively.

The liquid composition according to the invention has a weight content of allulose of at least 70 wt.-%, relative to the total weight of the liquid composition. Preferably, the liquid composition according to the invention has a weight content of allulose of at least 75 wt.-%, at least 80 wt.-%, at least 85 wt.-%, or at least 90 wt.-%, in each case relative to the total weight of the liquid composition.

Preferably, the liquid composition according to the invention has a weight content of allulose of not more than 99 wt.-%, not more than 98 wt.-%, not more than 97 wt.-%, not more than 96 wt.-%, not more than 95 wt.-%, not more than 94 wt.-%, not more than 93 wt.-%, not more than 92 wt.-%, not more than 91 wt.-%, not more than 90 wt.-%, not more than 89 wt.-%, not more than 88 wt.-%, not more than 87 wt.-%, not more than 86 wt.-%, not more than 85 wt.-%, not more than 84 wt.-%, not more than 83 wt.-%, not more than 82 wt.-%, not more than 81 wt.-%, not more than 80 wt.-%, not more than 79 wt.-%, not more than 78 wt.-%, not more than 77 wt.-%, not more than 76 wt.-%, not more than 75 wt.-%, not more than 74 wt.-%, not more than 73 wt.-%, not more than 72 wt.-%, not more than 71 wt.-%, or not more than 70 wt.-%, in each case relative to the total weight of the liquid composition.

Preferably, the liquid composition according to the invention has a weight content of allulose of not more than 99%, more preferably not more than 98%, still more preferably not more than 97%, yet more preferably not more than 96%, even more preferably not more than 95%, most preferably not more than 94%, and in particular not more than 93%, in each case of that weight content of allulose that would be contained in a fully saturated solution of allulose at ambient conditions.

The weight content of allulose that would be contained in a fully saturated solution of allulose at ambient conditions can be determined by simple routine experiments.

Besides allulose, the liquid composition according to the invention may comprise additional carbohydrates (saccharides), such as other monosaccharides, disaccharides or oligosaccharides. Preferred additional carbohydrates include but are not limited to glucose, fructose, sucrose, and mixtures thereof.

The liquid composition according to the invention has a weight content of allulose of at least 97 wt.-%, relative to the total content of all carbohydrates that are contained in the liquid composition.

Preferably, the liquid composition according to the invention has a weight content of allulose of at least 98 wt.-%, at least 99 wt.-%, or about 100 wt.-%, in each case relative to the total content of all carbohydrates that are contained in the liquid composition.

In a preferred embodiment, allulose is substantially the only carbohydrate that is contained in the liquid composition according to the invention.

In another preferred embodiment, the liquid composition according to the invention has a weight content of allulose of not more than 99 wt.-%, not more than 98 wt.-%, not more than 97 wt.-%, in each case relative to the total content of all carbohydrates that are contained in the liquid composition.

The brix value (degrees brix, symbol °Bx) is the sugar content of an aqueous solution. One degree Brix is 1 gram of sucrose in 100 grams of solution and represents the strength of the solution as percentage by mass. If the solution contains dissolved solids other than pure sucrose, then the °Bx only approximates the dissolved solid content. The brix value is determined by refractometry.

Preferably, the liquid composition according to the invention has a brix value of at least 50°, at least 51°, at least 52°, at least 53°, at least 54°, at least 55°, at least 56°, at least 57°, at least 58°, at least 59°, at least 60°, at least 61°, at least 62°, at least 63°, at least 64°, at least 65°, at least 66°, at least 67°, at least 68°, at least 69°, at least 70°, at least 71°, at least 72°, at least 73°, at least 74°, at least 75°, at least 76°, at least 77°, at least 78°, at least 79°, or at least 80°.

Preferably, the liquid composition according to the invention has a brix value of not more than 90°, not more than 89°, not more than 88°, not more than 87°, not more than 86°, not more than 85°, not more than 84°, not more than 83°, not more than 82°, not more than 81°, not more than 80°, not more than 79°, not more than 78°, not more than 77°, not more than 76°, not more than 75°, not more than 74°, not more than 73°, not more than 72°, not more than 71°, not more than 70°, not more than 69°, not more than 68°, not more than 67°, not more than 66°, not more than 65°, not more than 64°, not more than 63°, not more than 62°, not more than 61°, or not more than 60°.

In preferred embodiments, the liquid composition according to the invention has a brix value within the range of 50±30°, 50±25°, 50±20°, 50±15°, 50±10°, 50±5°, 60±30°, 60±25°, 60±20°, 60±15°, 60±10°, 60±5°, 70±30°, 70±25°, 70±20°, 70±15°, 70±10°, 70±5°, 80±30°, 80±25°, 80±20°, 80±15°, 80±10°, 80±5°, 90±30°, 90±25°, 90±20°, 90±15°, 90±10°, or 90±5°.

Preferably, the liquid composition according to the invention has a weight content of water of not more than 90 wt.-%, not more than 85 wt.-%, not more than 80 wt.-%, not more than 75 wt.-%, not more than 70 wt.-%, not more than 65 wt.-%, not more than 60 wt.-%, not more than 55 wt.-%, not more than 50 wt.-%, or not more than 45 wt.-%, or not more than 40 wt.-%, or not more than 35 wt.-%, or not more than 30 wt.-%, or not more than 25 wt.-%, or not more than 20 wt.-%, in each case relative to the total weight of the liquid composition.

Preferably, the liquid composition according to the invention has a weight content of water of at least 15 wt.-%, at least 20 wt.-%, at least 25 wt.-%, at least 30 wt.-%, at least 35 wt.-%, at least 40 wt.-%, at least 45 wt.-%, at least 50 wt.-%, at least 55 wt.-%, or at least 60 wt.-%, in each case relative to the total weight of the liquid composition.

The liquid composition according to the invention has a pH value within the range of 4.0±0.5. Preferably, the liquid composition according to the invention has a pH value of not more than 4.4, not more than 4.3, not more than 4.2, not more than 4.1, not more than 4.0, not more than 3.9, not more than 3.8, not more than 3.7, not more than 3.6, not more than 3.5.

Preferably, the liquid composition according to the invention has a pH value of at least 3.6, at least 3.7, at least 3.8, at least 3.9, at least 4.0, at least 4.1, at least 4.2, at least 4.3, at least 4.4, at least 4.5.

Preferably, the liquid composition according to the invention has a viscosity of at least 2.0 mPa·s, at least 2.5 mPa·s, at least 5.0 mPa·s, at least 7.5 mPa·s, at least 10 mPa·s, at least 12.5 mPa s, at least 15 mPa·s, at least 20 mPa·s, at least 25 mPa·s, at least 30 mPa·s, at least 35 mPa·s, at least 40 mPa·s, at least 45 mPa·s, at least 50 mPa·s, at least 55 mPa·s, at least 60 mPa·s, at least 65 mPa·s, at least 70 mPa·s, at least 75 mPa·s, or at least 80 mPa·s.

The liquid composition according to the invention has a viscosity of not more than 200 mPa s, measured by means of a rotary viscosimeter at 23 °C at a speed of 100 rpm. Preferably, the liquid composition according to the invention has a viscosity of not more than 190 mPa s, not more than 180 mPa s, not more than 170 mPa s, not more than 160 mPa s, not more than 150 mPa s, not more than 140 mPa·s, not more than 130 mPa·s, not more than 120 mPa·s, not more than 110 mPa·s, not more than 100 mPa·s, not more than 95 mPa·s, not more than 90 mPa·s, not more than 85 mPa·s, not more than 80 mPa·s, not more than 75 mPa·s, not more than 70 mPa·s, not more than 65 mPa·s, not more than 60 mPa·s, not more than 55 mPa·s, not more than 50 mPa·s, not more than 45 mPa·s, or not more than 40 mPa·s.

In preferred embodiments, the liquid composition according to the invention has a viscosity within the range of 60±50 mPa·s, 60±40 mPa·s, 60±30 mPa·s, 60±20 mPa·s, 80±50 mPa·s, 80±40 mPa·s, 80±30 mPa·s, 80±20 mPa·s, 100±50 mPa·s, 100±40 mPa·s, 100±30 mPa·s, 100±20 mPa·s, 120±50 mPa·s, 120±40 mPa·s, 120±30 mPa·s, 120±20 mPa s, 140±50 mPa·s, 140±40 mPa·s, 140±30 mPa·s, 140±20 mPa·s, 160±40 mPa·s, 160±30 mPa·s, 160±20 mPa·s, or 180±20 mPa·s.

The viscosity is measured by means of a rotary viscosimeter at 23 °C, at a speed of 100 rpm.

Preferably, the liquid composition according to the invention has a total weight content of carbohydrates (saccharides) other than allulose of
- not more than 20 wt.-%, not more than 10 wt.-%, not more than 5.0 wt.-%, not more than 4.5 wt.-%, not more than 4.0 wt.-%, not more than 3.5 wt.-%, not more than 3.0 wt.-%, not more than 2.5 wt.-%, not more than 2.0 wt.-%, not more than 1.5 wt.-%, not more than 1.0 wt.-%, or not more than 0.5 wt.-%, in each case relative to the total weight of the liquid composition; and/or
- at least 5.0 wt.-%, at least 10 wt.-%, at least 15 wt.-%, at least 20 wt.-%, at least 25 wt.-%, at least 30 wt.-%, in each case relative to the total weight of the liquid composition.

Preferably, the liquid composition according to the invention has a total content of carbohydrates (saccharides) including allulose of
- not more than 90 wt.-%, not more than 95 wt.-%, not more than 90 wt.-%, not more than 85 wt.-%, not more than 80 wt.-%, not more than 75 wt.-%, in each case relative to the total weight of the liquid composition; and/or
- at least 75 wt.-%, at least 80 wt.-%, in each case relative to the total weight of the liquid composition.

In a preferred embodiment, besides allulose the liquid composition according to the invention additionally comprises sucrose.

Preferably, the content of sucrose is
- at least 0.001 wt.-%, at least 0.005 wt.-%, at least 0.01 wt.-%, at least 0.05 wt.-%, at least 0.1 wt.-%, at least 0.2 wt.-%, at least 0.3 wt.-%, at least 0.4 wt.-%, or at least 0.5 wt.-%, at least 1.0 wt.-%, at least 2.5 wt.-%, at least 5.0 wt.-%, at least 7.5 wt.-%, at least 10 wt.-%, at least 12.5 wt.-%, at least 15 wt.-%, at least 17.5 wt.-%, at least 20 wt.-%, at least 22.5 wt.-%, or at least 25 wt.-%, in each case relative to the total weight of the liquid composition; and/or
- not more than 10 wt.-%, not more than 9.5 wt.-%, not more than 9.0 wt.-%, not more than 8.5 wt.-%, not more than 8.0 wt.-%, not more than 7.5 wt.-%, not more than 6.0 wt.-%, not more than 5.5
wt.-%, not more than 5.0 wt.-%, not more than 4.5 wt.-%, not more than 4.0 wt.-%, not more than 3.5 wt.-%, not more than 3.0 wt.-%, not more than 2.5 wt.-%, not more than 2.0 wt.-%, not more than 1.5 wt.-%, not more than 1.0 wt.-%, or not more than 0.5 wt.-%, in each case relative to the total weight of the liquid composition.

In a preferred embodiment, the sucrose is present in form of particles which essentially contain no allulose and/or wherein the allulose is present in form of particles which essentially contain no sucrose.

In a preferred embodiment, besides allulose the liquid composition according to the invention additionally comprises fructose.

Preferably, the content of fructose is
- at least 0.001 wt.-%, at least 0.005 wt.-%, at least 0.01 wt.-%, at least 0.05 wt.-%, at least 0.1 wt.-%, at least 0.2 wt.-%, at least 0.3 wt.-%, at least 0.4 wt.-%, or at least 0.5 wt.-%, at least 1.0 wt.-%, at least 2.5 wt.-%, at least 5.0 wt.-%, at least 7.5 wt.-%, at least 10 wt.-%, at least 12.5 wt.-%, at least 15 wt.-%, at least 17.5 wt.-%, at least 20 wt.-%, at least 22.5 wt.-%, or at least 25 wt.-%, in each case relative to the total weight of the liquid composition; and/or
- not more than 10 wt.-%, not more than 9.5 wt.-%, not more than 9.0 wt.-%, not more than 8.5 wt.-%, not more than 8.0 wt.-%, not more than 7.5 wt.-%, not more than 6.0 wt.-%, not more than 5.5 wt.-%, not more than 5.0 wt.-%, not more than 4.5 wt.-%, not more than 4.0 wt.-%, not more than 3.5 wt.-%, not more than 3.0 wt.-%, not more than 2.5 wt.-%, not more than 2.0 wt.-%, not more than 1.5 wt.-%, not more than 1.0 wt.-%, or not more than 0.5 wt.-%, in each case relative to the total weight of the liquid composition.

In a preferred embodiment, besides allulose the liquid composition according to the invention additionally comprises a sweetener and/or sugar substitute.

Preferably, the sweetener and/or sugar substitute is selected from the group consisting of acesulfame, aspartame, aspartame-acesulfame-salt, cyclamate, neohesperidine, neotame, saccharine, sucralose, stevioglycoside, especially stevioside, stevia, mogrosides, monkfruit, thaumatine, alitame, brazzeine, dulcine, hernandulcine, lugduname, monelline, pentadine, curculine, miraculine, osladine, perillartine, sorbitol, xylitol, mannitol, isomaltitol, isomaltulose, maltitol, lactitol, erythritol, and tagatose.

In a preferred embodiment, besides allulose the liquid composition according to the invention additionally comprises a monosaccharide or disaccharide or polysaccharide. The monosaccharide fructose and the disaccharide sucrose have been described above.

Preferably, the monosaccharide is selected from the group consisting of glucose, mannose and galactose.

Preferably, the disaccharide is selected from the group consisting of maltose, lactose and cellobiose.

Preferably, the polysaccharide is a starch, preferably maize starch or potato starch.

Preferably, the content of monosaccharide or disaccharide or polysaccharide is
- at least 0.001 wt.-%, at least 0.005 wt.-%, at least 0.01 wt.-%, at least 0.05 wt.-%, at least 0.1 wt.-%, at least 0.2 wt.-%, at least 0.3 wt.-%, at least 0.4 wt.-%, or at least 0.5 wt.-%, at least 1.0 wt.-%, at least 2.5 wt.-%, at least 5.0 wt.-%, at least 7.5 wt.-%, at least 10 wt.-%, at least 12.5 wt.-%, at least 15 wt.-%, at least 17.5 wt.-%, at least 20 wt.-%, at least 22.5 wt.-%, or at least 25 wt.-%, in each case relative to the total weight of the liquid composition; and/or
- not more than 10 wt.-%, not more than 9.5 wt.-%, not more than 9.0 wt.-%, not more than 8.5 wt.-%, not more than 8.0 wt.-%, not more than 7.5 wt.-%, not more than 6.0 wt.-%, not more than 5.5 wt.-%, not more than 5.0 wt.-%, not more than 4.5 wt.-%, not more than 4.0 wt.-%, not more than 3.5 wt.-%, not more than 3.0 wt.-%, not more than 2.5 wt.-%, not more than 2.0 wt.-%, not more than 1.5 wt.-%, not more than 1.0 wt.-%, or not more than 0.5 wt.-%, in each case relative to the total weight of the liquid composition.

In a preferred embodiment, besides allulose the liquid composition according to the invention additionally comprises an acid.

Preferably, the acid is an organic acid. Preferably, the organic acid is a carboxylic acid. Preferably, the carboxylic acid is a multicarboxylic acid. Preferably, the acid is selected from citric acid and tartaric acid.

Preferably, the content of the acid is
- at least 0.001 wt.-%, at least 0.005 wt.-%, at least 0.01 wt.-%, at least 0.05 wt.-%, at least 0.1 wt.-%, at least 0.2 wt.-%, at least 0.3 wt.-%, at least 0.4 wt.-%, at least 0.5 wt.-%, at least 0.75 wt.-%, at least 1.0 wt.-%, at least 15 wt.-%, at least 2.0 wt.-%, relative to the total weight of the liquid composition; and/or
- not more than 10 wt.-%, not more than 5.0 wt.-%, not more than 4.5 wt.-%, not more than 4.0 wt.-%, not more than 3.5 wt.-%, not more than 3.0 wt.-%, not more than 2.5 wt.-%, not more than 2.0 wt.-%, not more than 1.5 wt.-%, not more than 1.0 wt.-%, or not more than 0.5 wt.-%, relative to the total weight of the liquid composition.

In a preferred embodiment, the liquid composition according to the invention comprises essentially no carbohydrate other than allulose.

In a preferred embodiment, besides allulose the liquid composition according to the invention essentially contains
- no additional saccharides and/or
- no sweeteners and/or
- no antioxidants and/or
- no preservatives.

In a preferred embodiment, the liquid composition according to the invention essentially contains no hydroxymethyl furfural (HMF).

In a preferred embodiment, the liquid composition according to the invention essentially consists of allulose and water.

Another aspect of the invention relates to a container comprising the aqueous liquid composition according to the invention as described above. The volume of the container is not particularly limited.

Preferably, the container comprises the liquid composition according to the invention in form of a bulk material, preferably at least 10 kg, or at least 20 kg, or at least 30 kg, or at least 40 kg, or at least 50 kg, or at least 75 kg, or at least 100 kg of liquid composition.

Another aspect of the invention relates to the use of the liquid composition according to the invention as described above as a prebiotic or for rendering a foodstuff, a beverage or a feed for animals with prebiotic properties.

Another aspect of the invention relates to the use of the liquid composition according to the invention as described above for preparing a foodstuff, a beverage or a feed for animals.

Another aspect of the invention relates to a process for preparing a foodstuff or a beverage comprising the step of adding a liquid composition according to the invention as described above to an intermediate of the foodstuff, a beverage or a feed for animals.

All preferred embodiments of the liquid composition according to the invention as described above also analogously apply to the uses according to the invention and to the process according to the invention and are therefore not repeated hereinafter.

Preferably, the foodstuff, a beverage or a feed for animals according to the invention is solid, semi-solid or liquid composition.

Preferably, the foodstuff or beverage according to the invention is selected from foodstuffs and beverages.

Preferably, the foodstuff is selected from the group consisting of basic foods and prepared foods.

Preferred basic foods include but are not limited to breads, dairy products, eggs, legumes, edible plants, edible fungi, meat, edible nuts and seeds, cereals, seafood, and staple foods.

Preferred prepared foods include but are not limited to appetizers, condiments, confectionery, convenience foods, desserts, dips, pastes and spreads, dried foods, dumplings, fast food, fermented foods, halal food, kosher food, noodles, pies, salads, sandwiches, sauces, snack foods, soups, and stews.

Preferred foodstuffs are selected from the group consisting of basic foods and prepared foods. Preferred basic foods are selected from the group consisting of breads, dairy products, eggs, legumes, edible plants, edible fungi, meat, edible nuts and seeds, cereals, seafood, and staple foods. Preferred prepared foods are selected from the group consisting of appetizers, condiments, confectionery, convenience foods, desserts, dips, pastes and spreads, dried foods, dumplings, fast food, fermented foods, halal food, kosher food, noodles, pies, salads, sandwiches, sauces, snack foods, soups, and stews. In a preferred embodiment, the foodstuff is selected from the group consisting of food, functional food, food ingredients, dietary supplements, and feed.

Preferred beverages are selected from the group consisting of non-alcoholic drinks and alcoholic drinks. Preferred non-alcoholic drinks are selected from the group consisting of water, milk, tea, coffee, carbonated drinks, juice and juice drinks. Preferred alcoholic drinks are selected from the group consisting of beer, cider, wine, and spirits.

Particularly preferred foodstuffs and beverages include but are not limited to
- basic materials for beverages and drinks;
- formula diets;
- confectionaries;
- fruit preparations;
- sausages, cold cut and meat products;
- dairy products;
- bakery products (preferred sandwich bread, hamburger buns);
- and cereals preferred extruded cereals.

Examples of foodstuffs and beverages made with the liquid composition according to the invention include but are not limited to
- gels such as jelly, cream caramels, and yogurts;
- marmalade, jelly, fruit spread and jam;
- seasonings such as ketchup, mayonnaises, dressings, sauces, basting sauces, soups, and processed vegetable products; ketchup is particularly preferred;
- restorable foods such as curries, hashed beefs, meat sauces, stews, and soups;
- chilled foods;
- processed meat products such as hamburger steaks, bacons, sausages, salami sausages, and hams;
- fish paste products such as minced and steamed fish products, fish sausages, fish hams and sausages, and fried minced and steamed fish products;
- processed wheat products such as breads, raw noodles, dried noodles, macaronis, spaghettis, Chinese bun pastries, cake mixes, premixes, white sauces, and pastries for jiaozis and spring rolls;
- canned and bottled foods such as curries, sauces, soups, fish boiled in soy sauce, and jams;
- confectioneries such as candies, lozenges, tablet confectioneries, chocolates, biscuits, cookies, rice crackers, Japanese and Western cakes, unbaked cakes, snacks, sugar confectioneries, and cream caramels;
- deep-frozen foods;
- cooked and processed foods such as croquettes, jiaozis, and Chinese buns; and
- pastes such as vegetable pastes, minced meats, fruit pastes, and seafood pastes;
- dairy products such as ice creams, whipping creams, confluents, butters, yogurts, cheeses, and white sauces;
- processed oils and fats such as margarines, fat spreads, and shortenings;
- carbonated beverages such as colas, carbonated fruit beverages, alcoholic fruit beverages, fruit beverages mixed with dairy products, fruit juices, fruit-containing beverages;
- lactic acid beverages or milk beverages such as milk drinks, coffees, cow's milks, soy milks, cocoa milks, fruit milks, and yogurts; and
- tea beverages such as natural leaf teas, oolong teas, green teas, black teas.

Preferred beverages include but are not limited to non-alcoholic drinks and alcoholic drinks.

Preferred non-alcoholic drinks are selected from the group consisting of water, milk, cocoa milks, tea, coffee, carbonated drinks, juice and juice drinks.

Preferred alcoholic drinks are selected from the group consisting of beer, cider, wine, and spirits.

Preferred feeds are animal feeds such as concentrated feed preferably for feeding pets or livestock.

Preferred pets (domestic animals, farm animals) according to the invention include but are not limited to dogs, cats, mice, rats, rabbits, hamster, guinea pigs, birds, fish and the like. Preferred pets are selected from cats, dogs, rabbits, hamster and guinea pigs.

In a preferred embodiment, the animal feed is dry feed, preferably for pets, or wet feed, preferably for pets.

In another preferred embodiment, the animal feed is a liquid composition, preferably for pets.

Another aspect of the invention relates to a process for preparing a storage stable liquid allulose composition comprising the steps of
(a) providing an aqueous allulose composition;
(b) adjusting the dry solids content of the aqueous allulose composition such that it us from 50 wt.-% to 80 wt.-%;
(c) adjusting the allulose content of the aqueous allulose composition such that allulose is present in an amount of at least 80 wt.-% relative to the total dry solids content; and
(d) controlling the pH value of the aqueous allulose composition so that it is within the range of 4.0±0.5.

The following examples are not within the scope of the claims.

### Example 1 - a_{w}-value and viscosity of aqueous solutions:

The a_{w}-value and the viscosity of solutions prepared with allulose in distilled water were determined and compared with solutions of sucrose in distilled water. Further, the concentration of carbohydrates in solution (wTSr-value) was measured by means of a refractometer.

Aqueous solutions containing different amounts of allulose were prepared and analyzed. The amounts of allulose and the test results are displayed in the table below:

| ex. | allulose [g] | wTSr | aW | viscosity [mPa·s] | % | RPM |
|---|---|---|---|---|---|---|
| 1-1 | 5 | 4.76 | 0.969 | 1.85 | 9.6 | 200 |
| 1-2 | 10 | 9.49 | 0.977 | 2.06 | 11.9 | " |
| 1-3 | 15 | 14.13 | 0.967 | 2.31 | 13.3 | " |
| 1-4 | 20 | 18.82 | 0.965 | 2.62 | 15.1 | " |
| 1-5 | 25 | 23.78 | 0.956 | 2.98 | 17.2 | " |
| 1-6 | 30 | 28.51 | 0.946 | 3.52 | 20.3 | " |
| 1-7 | 35 | 33.24 | 0.944 | 4.46 | 25.7 | " |
| 1-8 | 40 | 37.97 | 0.921 | 5.44 | 31.4 | " |
| 1-9 | 45 | 42.71 | 0.905 | 7.35 | 42.4 | " |
| 1-10 | 50 | 47.44 | 0.887 | 11.90 | 68.9 | " |
| 1-11 | 55 | 52.16 | 0.867 | 16.10 | 46.4 | 100 |
| 1-12 | 60 | 56.85 | 0.838 | 26.40 | 76.3 | " |
| 1-13 | 65 | 61.52 | 0.803 | 53.40 | 77.0 | 50 |

Aqueous solutions containing different amounts of sucrose were prepared and analyzed. The amount of sucrose and the test results are displayed in the table below:

| ex. | sucrose [g] | wTSr | aW | viscosity [mPa·s] | % | RPM |
|---|---|---|---|---|---|---|
| 1-14 | 5 | 5.17 | 0.981 | 1.77 | 10.20 | 200 |
| 1-15 | 10 | 9.99 | 0.982 | 2.03 | 11.70 | " |
| 1-16 | 15 | 15.01 | 0.983 | 2.17 | 12.50 | " |
| 1-17 | *20 | 20.00 | 0.986 | 2.57 | 14.90 | " |
| 1-18 | 25 | 24.99 | 0.977 | 2.74 | 15.70 | " |
| 1-19 | 30 | 30.00 | 0.974 | 3.50 | 20.20 | " |
| 1-20 | 35 | 34.93 | 0.974 | 4.75 | 27.40 | " |
| 1-21 | 40 | 39.95 | 0.971 | 6.92 | 39.90 | " |
| 1-22 | 45 | 44.98 | 0.962 | 10.70 | 61.50 | " |
| 1-23 | 50 | 49.99 | 0.924 | 17.10 | 49.30 | 100 |
| 1-24 | 55 | 54.96 | 0.906 | 31.20 | 45.00 | 50 |
| 1-25 | 60 | 60.03 | 0.885 | 67.30 | 96.80 | " |
| 1-26 | 65 | 64.23 | 0.863 | not measurable | not measurable | |

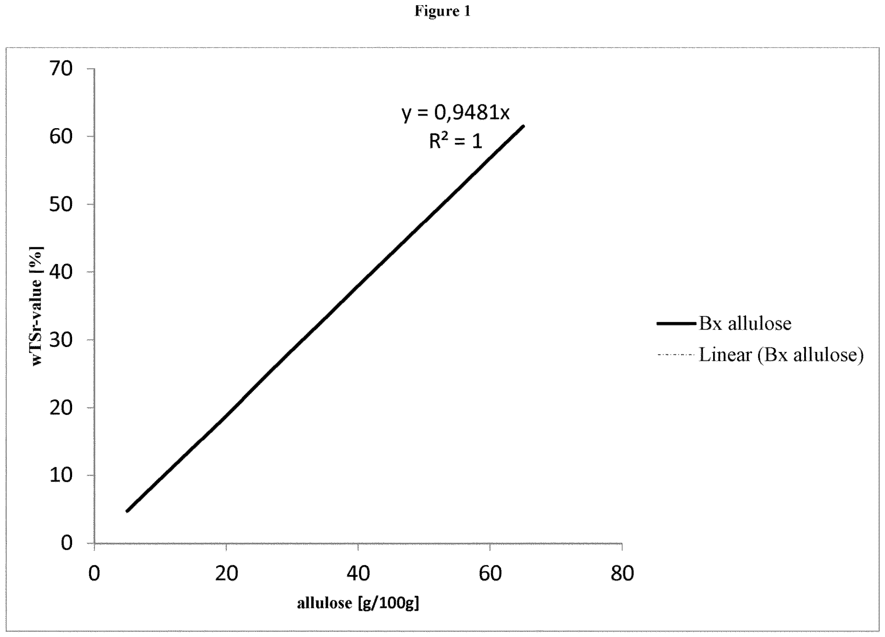

The results of the measurements of the concentration of carbohydrates in the solution (wTSr - values) are displayed in Figure 1.

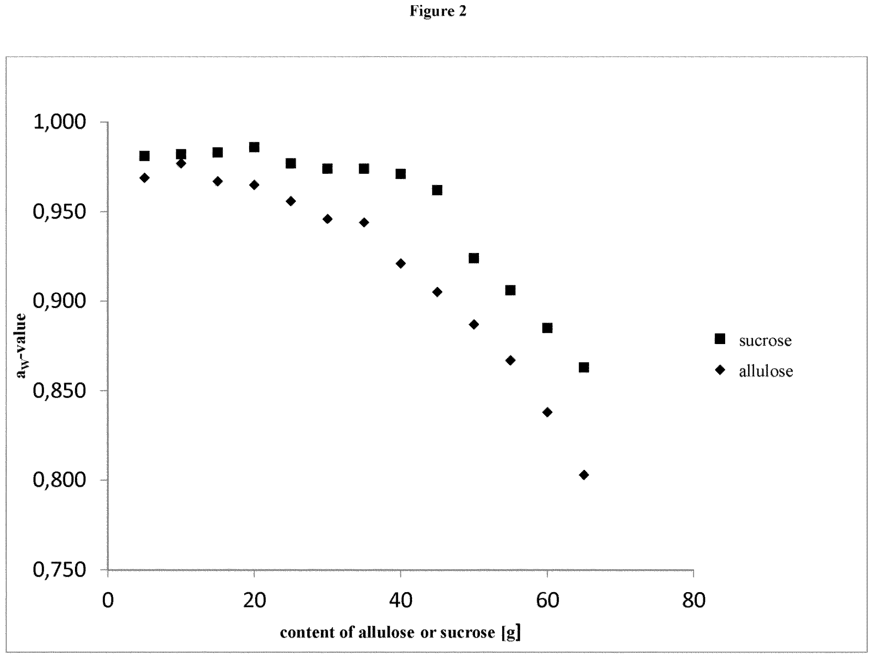

The results of the measurements of the a_{w}-values of the solutions containing distilled water and allulose, or sucrose respectively, as a function of the content of allulose, or sucrose respectively, are displayed in Figure 2.

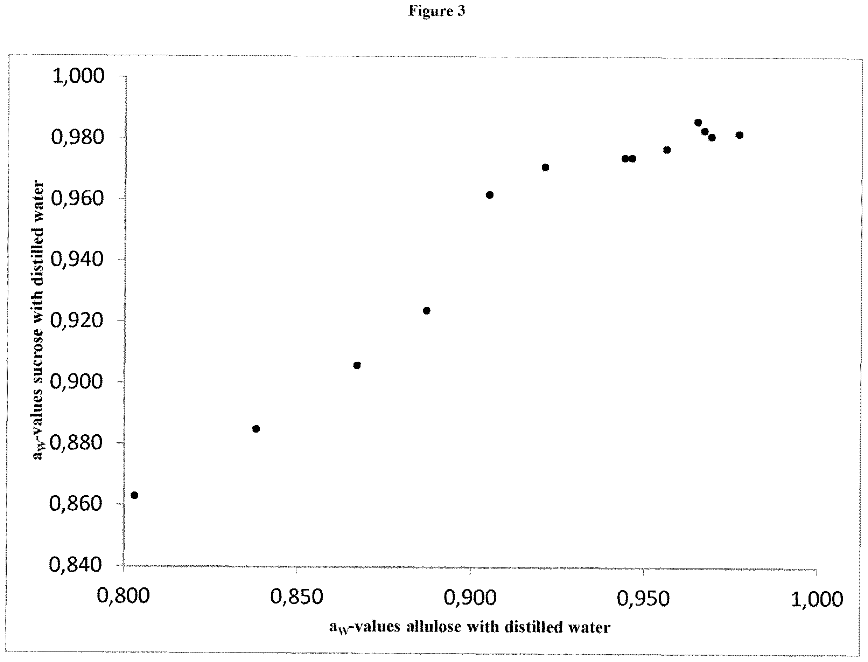

The results of the measurements of the a_{w}-values of the solutions containing distilled water and sucrose, as a function of the a_{w}-values of the solutions containing distilled water and allulose, are displayed in Figure 3.

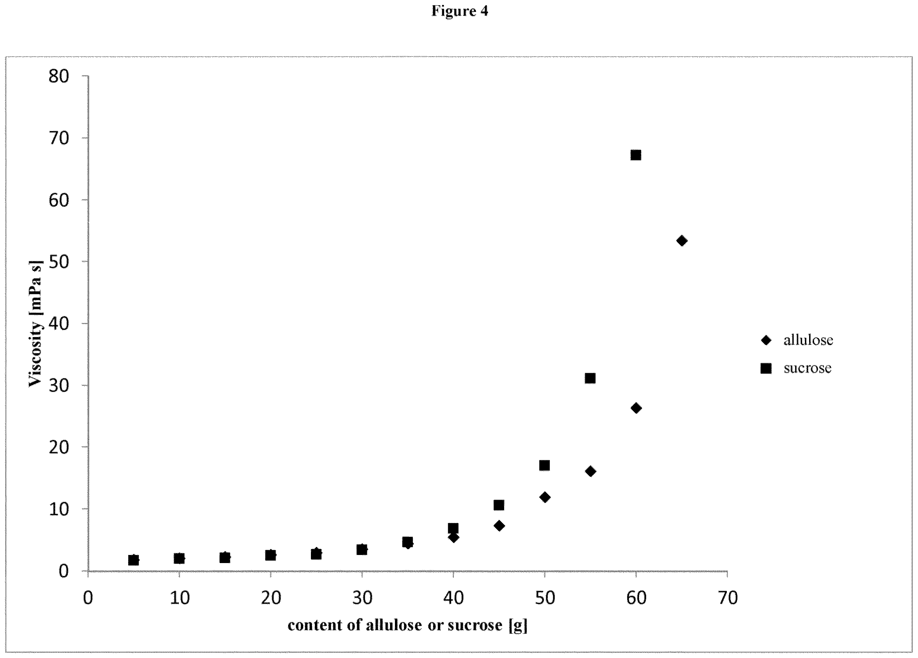

The results of the viscosity-measurements of the solutions containing distilled water and allulose, or sucrose respectively, as a function of the content of allulose, or sucrose respectively, are displayed in Figure 4.

It becomes clear from the experimental data that the solutions containing water and allulose according to the invention had a lower a_{w}-value than solutions containing water and the same amount of sucrose. Especially solutions with a high concentration of more than 30 g of allulose had a considerable lower water activity than solutions containing the respective same amount of sucrose.

Further, the experimental data show that solutions containing water and allulose had a lower viscosity compared to solutions containing water and the same amount of sucrose. Especially solutions with a high concentration of more than 45 g of allulose had a much lower viscosity than solutions containing the respective same amount of sucrose.

A lower viscosity at the same weight concentration is often advantageous and thus desirable, as it facilitates processing of liquid compositions.

### Example 2 - a_{w}-value and viscosity of cold aqueous solutions of allulose in apple juice:

The a_{w}-value and the viscosity of cold solutions of allulose in apple juice were determined and compared with solutions of sucrose in apple juice. Further, the concentration of carbohydrates in the solution (wTSr-value) was measured by means of a refractometer.

Cold solutions containing different amounts of apple juice and different amounts of allulose were prepared and analyzed. The respective amounts and the test results are displayed in the table below:

| ex. | allulose [g] | apple juice [g] | wTSr | aW | viscosity [mPa·s] | % | RPM |
|---|---|---|---|---|---|---|---|
| 2-1 | 53.33 | 106.67 | 38.93 | 0.921 | 5.65 | 32.6 | 200 |
| 2-2 | 60.00 | 100.00 | 42.43 | 0.908 | 6.78 | 39.1 | 200 |
| 2-3 | 65.88 | 94.12 | 45.47 | 0.897 | 8.17 | 47.1 | 200 |
| 2-4 | 71.11 | 88.89 | 48.21 | 0.888 | 10.4 | 60.1 | 200 |
| 2-5 | 75.79 | 84.21 | 50.65 | 0.876 | 13.9 | 80.3 | 200 |
| 2-6 | 80.00 | 80.00 | 52.85 | 0.864 | 15.2 | 43.8 | 100 |

Cold solutions containing different amounts of apple juice and different amounts of sucrose were prepared and analyzed. The respective amounts and the test results are displayed in the table below:

| ex. | sucrose [g] | apple juice [g] | wTSr | aW | viscosity [mPa·s] | % | RPM |
|---|---|---|---|---|---|---|---|
| 2-7 | 53.33 | 106.67 | 40.64 | 0.946 | 7.2 | 41.5 | 200 |
| 2-8 | 60.00 | 100.00 | 43.94 | 0.937 | 9.8 | 56.5 | 200 |
| 2-9 | 65.88 | 94.12 | 47.10 | 0.929 | 14.6 | 84.0 | 200 |
| 2-10 | 71.11 | 88.89 | 49.95 | 0.920 | 18 | 52.1 | 100 |
| 2-11 | 75.79 | 84.21 | 53.10 | 0.910 | 23.8 | 68.7 | 100 |
| 2-12 | 80.00 | 80.00 | 55.18 | 0.902 | 29.9 | 86.1 | 100 |

It becomes clear from the experimental data that solutions containing apple juice and allulose had a lower a_{w}-value than solutions containing apple juice and the same amount of sucrose. Especially solutions with a higher carbohydrate concentration of more than 65 g of allulose had a considerable lower a_{w}-value than solutions containing the respective same amount of sucrose.

Further, the experimental data shows that solutions containing apple juice and allulose had a lower viscosity compared to solutions containing water and the respective same amount of sucrose. Especially solutions with a high carbohydrate concentration of allulose had a much lower viscosity than solutions containing apple juice and sucrose.

### Example 3 - a_{w}-value and viscosity of cooked aqueous solutions of allulose in apple juice:

The a_{w}-value and the viscosity of cooked solutions of allulose in apple juice were determined and compared with solutions of sucrose in apple juice. Further, the concentration of carbohydrates in the solution (wTSr-value) was measured by means of a refractometer.

Different amounts of apple juice and different amounts of allulose were mixed and subsequently cooked for four minutes. Citric acid was added just before cooking. The thus obtained solutions were analyzed. The respective amounts and the test results are displayed in the table below:

| ex. | citric acid [g] | allulose [g] | apple juice [g] | wTSr | aW | viscosity [mPa·s] | % | RPM | pH-value | effective boiling time |
|---|---|---|---|---|---|---|---|---|---|---|
| 3-1 | 0.53 | 53.33 | 106.67 | 39.46 | 0.918 | 6.24 | 18.0 | 100 | 3.06 | 01:53 |
| 3-2 | 0.60 | 60.00 | 100.00 | 43.06 | 0.904 | 7.84 | 22.6 | 100 | 3.00 | 01:53 |
| 3-3 | 0.66 | 65.88 | 94.12 | 46.02 | 0.894 | 9.71 | 28.0 | 100 | 2.98 | 01:57 |
| 3-4 | 0.71 | 71.11 | 88.89 | 48.78 | 0.883 | 12.4 | 35.7 | 100 | 2.93 | 01:56 |
| 3-5 | 0.76 | 75.79 | 84.21 | 51.49 | 0.871 | 15.6 | 45.1 | 100 | 2.92 | 01:57 |
| 3-6 | 0.80 | 80.00 | 80.00 | 53.60 | 0.861 | 19.7 | 42.6 | 75 | 2.89 | 01:54 |

Different amounts of apple juice and different amounts of sucrose were mixed and subsequently cooked for four minutes. Citric acid was added just before cooking. The thus obtained solutions were analyzed. The respective amounts and the test results are displayed in the table below:

| ex. | citric acid [g] | sucrose [g] | apple juice [g] | wTSr | aW | viscosity [mPa·s] | % | RPM | pH-value | effective boiling time |
|---|---|---|---|---|---|---|---|---|---|---|
| 3-7 | 0.53 | 53.33 | 106.67 | 41.61 | 0.927 | 8.18 | 23.6 | 100 | 3.01 | 01:50 |
| 3-8 | 0.60 | 60.00 | 100.00 | 45.50 | 0.921 | 11.0 | 31.6 | 100 | 2.96 | 02:00 |
| 3-9 | 0.66 | 65.88 | 94.12 | 48.78 | 0.91 | 14.6 | 42.1 | 100 | 2.91 | 02:00 |
| 3-10 | 0.71 | 71.11 | 88.89 | 51.99 | 0.895 | 20.0 | 57.7 | 100 | 2.86 | 02:00 |
| 3-11 | 0.76 | 75.79 | 84.21 | 54.41 | 0.885 | 26.1 | 75.4 | 100 | 2.82 | 02:05 |
| 3-12 | 0.80 | 80.00 | 80.00 | 56.87 | 0.872 | 36.5 | 78.9 | 75 | 2.79 | 02:00 |

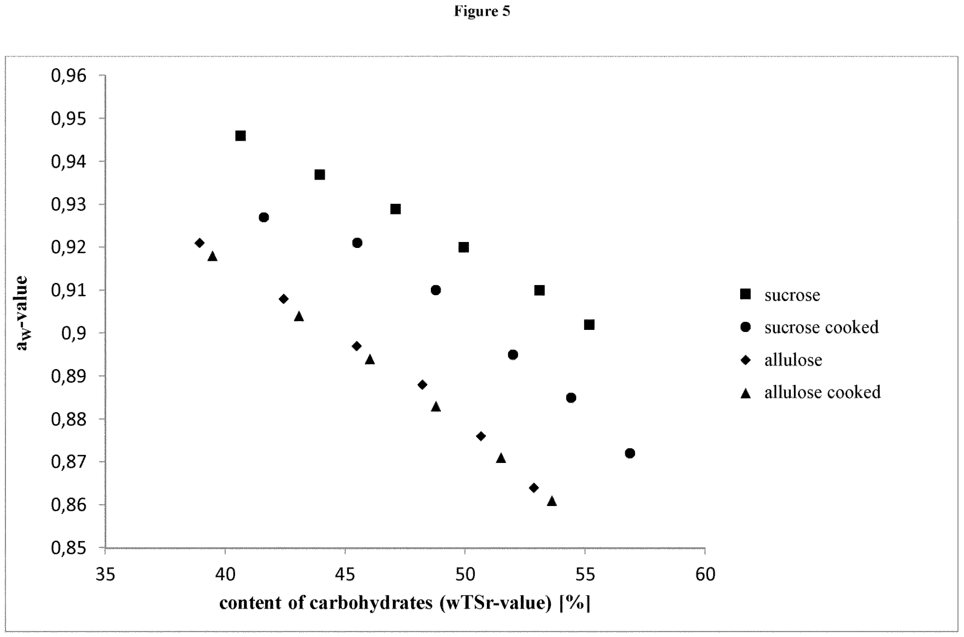

The results of the measurements of the a_{w}-values of the cold and cooked solutions containing apple juice and allulose, or sucrose respectively, as a function of the concentration of carbohydrates in the solution (wTSr -values) are displayed in Figure 5.

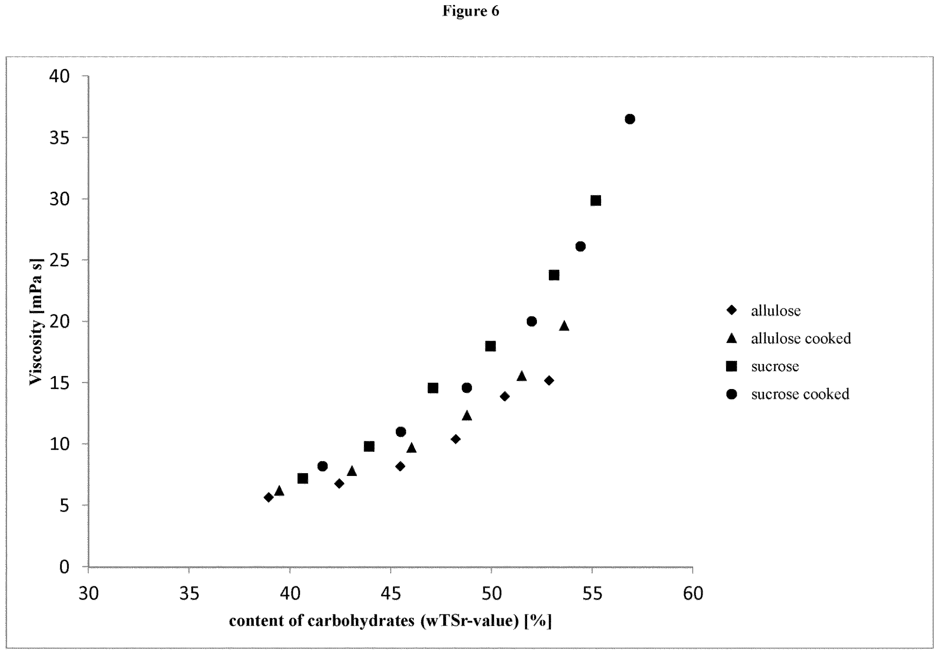

The viscosity-values of the solutions containing apple juice and allulose, or sucrose respectively, before and after cooking as a function of the concentration of carbohydrates in the solution (wTSr -values) are displayed in Figure 6.

Like the cold solutions, also the cooked solutions containing apple juice and allulose had lower a_{w}-value than solutions containing apple juice and the same amount of sucrose. Also the wTSr-value was lower in the solutions containing allulose compared to the solutions containing the respective same amount of sucrose.

Further, the experimental data shows that also the cooked solutions containing apple juice and allulose had a lower viscosity compared to solutions containing water and the respective same amount of sucrose. Especially solutions with a high carbohydrate concentration of allulose had a much lower viscosity than solutions containing apple juice and sucrose.

### Example 4 - storage stability:

Aqueous allulose solutions comprising 93 wt.-% of allulose relative to the total dry solids content and comprising 71 wt.-% or 77 wt.-% dry solids content are subjected to different pH values at different dry solids contents and different temperatures. Sealed sample containers are placed into different temperature ovens at 40 °C and 50 °C. Extracts from each of the samples are periodically removed from each oven. Samples are chilled quickly in an ice bath and analyzed for carbohydrate composition, color and pH value.

At 40 °C after 11 days and 19 days, respectively:

| pH | dry solids content [wt. -] | acid [60 ppm] | rel. change | | |
|---|---|---|---|---|---|
| | | | pH (19 d) | purity allulose (11 d) | color (11 d) [BHC] |
| 3.4 | 77 | - | ±0 | -2.3% | +0.6 |
| 3.4 | 71 | - | ±0 | -0.9% | +0.3 |
| 3.6 | 77 | - | -0.1 | -0.4% | +0.6 |
| 4.0 | 77 | - | -0.15 | -0.4% | +1.2 |
| 4.0 | 77 | citric acid | -0.1 | -0.25% | +1.5 |
| 4.7 | 77 | - | -0.4 | -0.2% | +2.5 |

At 50 °C after 11 days and 19 days, respectively:

| pH | dry solids content [wt. -] | acid [60 ppm] | rel. change | | |
|---|---|---|---|---|---|
| | | | pH (19 d) | purity allulose (11 d) | color (11 d) [BHC] |
| 3.4 | 77 | - | -0.2 | -5.0% | +3.0 |
| 3.4 | 71 | - | -0.2 | -3.0% | +2.8 |
| 3.6 | 77 | - | -0.2 | -3.6% | +3.0 |
| 4.0 | 77 | - | -0.4 | -1.9% | +4.6 |
| 4.0 | 77 | citric acid | -0.4 | -1.6% | +4.6 |
| 4.7 | 77 | - | -0.8 | -0.8% | +7.5 |

The pH value drops over the course of the experiments. The decrease in pH value is more pronounced in samples starting at higher pH, and the pH drops faster at higher temperature. The sample with only a slightly lower dry solid content, (71 wt.-% vs. 77 wt.-%) starting at pH 3.37 shows much less allulose loss than its equivalent pH sample at 77 wt.-% dry solids content. High pH, longer time and high temperature increase the color formation. By increasing the pH it is possible to mitigate the allulose content loss, however there is an upper limit bound by increasing color in the final product.

### Example 5 - crystallization stability:

Aqueous allulose solutions are prepared at different dry solids contents and equilibrated at different temperatures. These samples are seeded with ~0.1 % crystalline allulose and crystallization is monitored visually and by change in dry solids after 1 month of storage:

Relative change in dry solids content after storage at 4 °C:

| dry solids content | 2 weeks | 4 weeks |
|---|---|---|
| 50 wt.-% | 0 | 0 |
| 60 wt.-% | 0 | 0 |
| 71 wt.-% | 0 | 0 |
| 77 wt.-% | 5 | 5 |
| 85 wt.-% | 9 | 9 |

Relative change in dry solids content after storage at 15 °C:

| dry solids content | 2 weeks | 4 weeks |
|---|---|---|
| 50 wt.-% | 0 | 0 |
| 60 wt.-% | 0 | 0 |
| 71 wt.-% | 0 | 0 |
| 77 wt.-% | 2 | 2 |
| 85 wt.-% | 7 | 8 |

Relative change in dry solids content after storage at 25 °C:

| dry solids content | 2 weeks | 4 weeks |
|---|---|---|
| 50 wt.-% | 0 | 0 |
| 60 wt.-% | 0 | 0 |
| 71 wt.-% | 0 | 0 |
| 77 wt.-% | 0 | 0 |
| 85 wt.-% | 5 | 6 |

A change in dry solids content greater than 0 indicates crystallization, and a larger number indicates a larger amount of crystallization.

## Claims

1. An aqueous liquid composition comprising allulose,
wherein the weight content of allulose is at least 70 wt.-%, relative to the total weight of the liquid composition;
wherein the weight content of allulose is at least 97 wt.-%, relative to the total content of all carbohydrates that are contained in the liquid composition;
wherein the liquid composition has a pH value within the range of 4.0±0.5; and
wherein the liquid composition has a viscosity of not more than 200 mPa·s, measured by means of a rotary viscosimeter at 23 °C at a speed of 100 rpm.

2. The liquid composition according to claim 1, wherein the weight content of allulose is at least 98 wt.-%, relative to the total content of all carbohydrates that are contained in the liquid composition.

3. The liquid composition according to any of the preceding claims, which has a coloring capacity of not more than 100 EBC.

4. The liquid composition according to any of the preceding claims, which has a weight content of undissolved material of not more than 10 wt.-%, relative to the total weight of the liquid composition.

5. The liquid composition according to any of the preceding claims, which comprises
- no sweeteners and/or
- no antioxidants and/or
- no preservatives and/or
- no hydroxymethyl furfural.

6. The liquid composition according to any of the preceding claims, which additionally comprises an acid.

7. The liquid composition according to claim 6, wherein the content of the acid is at least 0.01 wt.-%, relative to the total weight of the liquid composition.

8. The liquid composition according to claim 6 or 7, wherein the content of the acid is not more than 2.0 wt.-%, relative to the total weight of the liquid composition.

9. Use of an aqueous liquid composition according to any of claims 1 to 8 for preparing a foodstuff, a beverage, or an animal feed.

10. The use according to claim 9, wherein the foodstuff or beverage is selected from basic materials for beverages and drinks; formula diets; confectionaries; fruit preparations; sausages, cold cut and meat products; dairy products; bakery products; and cereals.

11. A process for preparing a foodstuff, beverage or an animal feed comprising the step of adding an aqueous liquid composition according to any of claims 1 to 8 to an intermediate of the foodstuff, the beverage, or the animal feed.

12. The process according to claim 11, wherein the foodstuff or beverage is selected from basic materials for beverages and drinks; formula diets; confectionaries; fruit preparations; sausages, cold cut and meat products; dairy products; bakery products; and cereals.

## Patentansprüche

1. Eine wässrige flüssige Zusammensetzung, die Allulose umfasst,
wobei der Gewichtsanteil der Allulose mindestens 70 Gew.-%, bezogen auf das Gesamtgewicht der flüssigen Zusammensetzung, beträgt;
wobei der Gewichtsanteil an Allulose mindestens 97 Gew.-% beträgt, bezogen auf das Gesamtgewicht aller Kohlenhydrate, die in der flüssigen Zusammensetzung enthalten sind;
wobei die flüssige Zusammensetzung einen pH-Wert im Bereich von 4,0±0,5 aufweist; und
wobei die flüssige Zusammensetzung eine Viskosität von nicht mehr als 200 mPa.s aufweist, gemessen mit einem Rotationsviskosimeter bei 23 °C und einer Geschwindigkeit von 100 U/min.

2. Die flüssige Zusammensetzung nach Anspruch 1, wobei der Gewichtsanteil an Allulose mindestens 98 Gew.-% beträgt, bezogen auf das Gesamtgewicht aller Kohlenhydrate, die in der flüssigen Zusammensetzung enthalten sind.

3. Die flüssige Zusammensetzung nach einem der vorhergehenden Ansprüche, die eine Färbekapazität von nicht mehr als 100 EBC aufweist.

4. Die flüssige Zusammensetzung nach einem der vorhergehenden Ansprüche, die einen Gewichtsanteil an ungelöstem Material von nicht mehr als 10 Gew.-%, bezogen auf das Gesamtgewicht der flüssigen Zusammensetzung, aufweist.

5. Die flüssige Zusammensetzung nach einem der vorhergehenden Ansprüche, die Folgendes umfasst
- keine Süßungsmittel und/oder
- keine Antioxidantien und/oder
- keine Konservierungsstoffe und/oder
- kein Hydroxymethylfurfural.

6. Die flüssige Zusammensetzung nach einem der vorhergehenden Ansprüche, die zusätzlich eine Säure enthält.

7. Die flüssige Zusammensetzung nach Anspruch 6, wobei der Gehalt der Säure mindestens 0,01 Gew.-%, bezogen auf das Gesamtgewicht der flüssigen Zusammensetzung, beträgt.

8. Die flüssige Zusammensetzung nach Anspruch 6 oder 7, wobei der Gehalt der Säure nicht mehr als 2,0 Gew.-%, bezogen auf das Gesamtgewicht der flüssigen Zusammensetzung, beträgt.

9. Verwendung einer wässrigen flüssigen Zusammensetzung nach einem der Ansprüche 1 bis 8 zur Herstellung eines Lebensmittels, eines Getränks oder eines Tierfutters.

10. Die Verwendung nach Anspruch 9, **dadurch gekennzeichnet, dass** das Lebensmittel oder Getränk ausgewählt wird aus Grundstoffen für Getränke und Drinks, Diätnahrung, Süßwaren, Fruchtzubereitungen, Wurst-, Aufschnitt- und Fleischwaren, Milchprodukten, Backwaren und Cerealien.

11. Verfahren zur Herstellung eines Lebensmittels, eines Getränks oder eines Tierfutters, umfassend den Schritt der Zugabe einer wässrigen flüssigen Zusammensetzung nach einem der Ansprüche 1 bis 8 zu einem Zwischenprodukt des Lebensmittels, des Getränks oder des Tierfutters.

12. Das Verfahren nach Anspruch 11, wobei das Lebensmittel oder Getränk ausgewählt wird aus Grundstoffen für Getränke und Drinks, Diätnahrung, Süßwaren, Fruchtzubereitungen, Wurst-, Aufschnitt- und Fleischwaren, Molkereiprodukten, Backwaren und Cerealien.

## Revendications

1. Composition liquide aqueuse comprenant de l'allulose,
dans laquelle la teneur en poids d'allulose est d'au moins 70 % en poids, par rapport au poids total de la composition liquide ; dans laquelle la teneur en poids d'allulose est d'au moins 97 % en poids, par rapport à la teneur totale de tous les glucides contenus dans la composition liquide ;
dans laquelle la composition liquide a une valeur de pH comprise dans la plage de 4,0±0,5 ; et
dans laquelle la composition liquide a une viscosité de pas plus de 200 mPa·s, mesurée au moyen d'un viscosimètre rotatif à 23 °C à une vitesse de 100 tr/min.

2. Composition liquide selon la revendication 1, dans laquelle la teneur en poids d'allulose est d'au moins 98 % en poids, par rapport à la teneur totale de tous les glucides contenus dans la composition liquide.

3. Composition liquide selon l'une quelconque des revendications précédentes, qui a une capacité de coloration de pas plus de 100 EBC.

4. Composition liquide selon l'une quelconque des revendications précédentes, qui a une teneur en poids de matière non dissoute de pas plus de 10 % en poids, par rapport au poids total de la composition liquide.

5. Composition liquide selon l'une quelconque des revendications précédentes, qui est exempte de
- édulcorants et/ou
- antioxydants et/ou
- conservateurs et/ou
- hydroxyméthylfurfural.

6. Composition liquide selon l'une quelconque des revendications précédentes, qui comprend également un acide.

7. Composition liquide selon la revendication 6, dans laquelle la teneur de l'acide est d'au moins 0,01 % en poids, par rapport au poids total de la composition liquide.

8. Composition liquide selon la revendication 6 ou 7, dans laquelle la teneur de l'acide n'est pas supérieure à 2,0 % en poids, par rapport au poids total de la composition liquide.

9. Utilisation d'une composition liquide aqueuse selon l'une quelconque des revendications 1 à 8 pour préparer un aliment, une boisson ou un aliment pour animaux.

10. Utilisation selon la revendication 9, dans laquelle l'aliment ou la boisson est choisi parmi des matières de base pour les boissons ; les formules de perte de poids ; les confiseries ; les préparations à base de fruits ; les saucisses, la charcuterie et les produits à base de viande ; les produits laitiers ; les produits de boulangerie ; et les céréales.

11. Procédé de préparation d'un aliment, d'une boisson ou d'un aliment pour animaux comprenant l'étape d'ajout d'une composition liquide aqueuse selon l'une quelconque des revendications 1 à 8 à un produit semi-fini de l'aliment, de la boisson ou de l'aliment pour animaux.

12. Procédé selon la revendication 11, dans lequel l'aliment ou la boisson est choisi parmi des matières de base pour les boissons ; les formules de perte de poids ; les confiseries ; les préparations à base de fruits ; les saucisses, la charcuterie et les produits à base de viande ; les produits laitiers ; les produits de boulangerie ; et les céréales.
